# EUROPEAN PATENT SPECIFICATION

(11) **EP 1 181 065 B1**
(45) Date of publication and mention of the grant of the patent: **30.07.2003**
(21) Application number: 99922157.5
(22) Date of filing: 03.05.1999
(51) Int. Cl.: A61M 5/14, A61M 5/38

(54) **INFUSION APPARATUS**
INFUSIONSVORRICHTUNG
INFUSEUR

(43) Date of publication of application: 27.02.2002
(73) Proprietor: BD Infusion Therapy GmbH, 69126 Heidelberg (DE)
(72) Inventor: ZECHENDORF, Klaus, 94447 Plattling (DE); MACHT, Wolfgang, 94447 Plattling (DE); KIRSCHNER, Gerhard, D-94034 Passau (DE); HUBER, Antje, D-91207 Lauf (DE)
(74) Representative: Dörries, Hans Ulrich, Dr.
(86) International application number: EP9902996
(87) International publication number: WO00066200

(56) References cited:
- EP-A- 0 001 114
- EP-A- 0 653 217
- WO-A-96/29104
- WO-A-98/46291
- GB-A- 2 044 620
- US-A- 4 173 222
- US-A- 4 413 990

## Description

The invention relates to an infusion apparatus in accordance with the preamble of patent claim 1.

Such infusion apparatuses may be used to introduce infusion solutions directly into a vein of a patient. Since, for many patients, a plurality of infusions frequently have to be administered one after the other, the requirement arises of being able to use an infusion apparatus used for one infusion also for further subsequent infusions. The transition from one infusion to the next takes place in such a way that, after the end of the first infusion, the infusion bottle is removed from the lance of the infusion apparatus, and a new bottle is placed onto the lance.

A problem with this replacement lies in the fact that, after an infusion bottle has run empty, and in particular after it has been removed from the lance, air can enter into the tube extending from the drip chamber to the patient. The air must then first be removed before the start of the subsequent infusion, if the danger of an air embolism in the patient is to be avoided. Even if no air enters into the tube when the infusion bottle has run empty, this may occur during an initial pumping procedure which is effected after a full infusion bottle has been set in place.

Since air, having once entered into the tube, can only be removed from the, latter with difficulty, the emptying of the infusion bottle must be observed by a supervisor, so that the tube clamp within the tube, which is preferably formed as a roll clamp, can be closed prior to complete discharge of the infusion solution from the drip chamber, and hence prior to air entering the tube.

Since such supervision cannot be expected, in particular in view of the shortage of personnel and the frequently hectic environment in hospitals, attempts have been made to prevent the entry of air into the tube, after the emptying of the infusion bottle by specific mechanisms.

It is already known from WO 96/34565 to incorporate a valve for this purpose into the lancing part or into the lance, which automatically closes after the infusion bottle has emptied, and can be opened again after the puncturing of a new infusion bottle. In this way care is taken that a certain liquid level is always maintained in the drip chamber after the infusion bottle has run empty, so that the danger of entry of air into the tube between the patient and the drip chamber is effectively avoided.

A problem with this known solution is, however, the requirement for a valve with a movable valve member, which must be lighter than the infusion solution, whereby the choice of material for the movable element is considerably restricted. Furthermore, the systems which operate with a valve are to be regarded as very unstable, because the buoyancy forces during continuing infusion are very small in comparison to the flow forces, and also because the valve member tends to stick due to adhesion forces to an empty infusion bottle so that the movable valve member is simply difficult to control with respect to its opening and closing movement Accordingly, undesired closing or opening of the valve repeatedly occurs.

A further system is known from WO96/29104 in which two filters are provided, one at each end of the drip chamber. Each filter serves to prevent latex particles and other contaminants from passing into the drip chamber as well as on into the tube. The filters further serve to prevent air from passing through the drip chamber in the down stream direction. The system described is intended to be primed for use and then transported. For this purpose the filters are provided in order to ensure that air may not pass into the drip chamber or from the drip chamber into the tube. The system is only intended for one-time use and therefore is not intended to provide for means to stop the flow of liquid downstream, even more means are provided to ensure that the flow downstream is always ensured as long as liquid is present in the system.

GB-A-2 044 620 discloses an infusion apparatus in accordance with the preamble of claim 1.

The object of the present invention is to provide an infusion apparatus of the initially described type which achieves a blockage of the flow of infusion liquid when sufficient liquid is still remaining in the drip chamber in order to prevent the entry of air into the tube and where the drip chamber is located at a height above the patient which corresponds to the conditions present in an actual hospital environment. Furthermore means are provided to make manual venting easier.

In order to satisfy this object there are provided the features of the characterizing part of claim 1.

A concept also used in the invention is thus to be seen in the fact that the adhesive forces present in a filter having a particular porosity, a particular hydrophilic coating and being mounted inside the drip chamber in a specific fashion are exploited in order to produce a barrier for air standing under atmospheric pressure, which would conventionally, i.e. without the filter of the invention, exert a pressure on the liquid in the drip chamber, which, with a corresponding height of the drip chamber above the patient and emptying of the infusion bottle, would not be capable of effecting a blockage of the flow of liquid into the tube and ultimately would also convey air into the tube.

Accordingly, the filter of the present invention arranged at a suitable position clearly above the base of the drip chamber represents, up to a certain pressure difference between the top and bottom side of the filter, a blockage for the passage of air through the filter which in return causes sufficient liquid to remain in the drip chamber once the infusion bottle is empty. The blockage is a result of liquid remaining in the pores of the filter and therefore preventing the passage of air through the pores.

In order to prevent air from entering the tube and then reaching the patient it is preferred to have the blockage of the flow of liquid occur at a point in time when a sufficient level of liquid still remains above the base of the drip chamber.

The venting of the drip chamber, in particular when introducing the first infusion for priming the infusion apparatus, but also when replacing the infusion bottle and restarting the flow of liquid, preferably takes place by squeezing together the flexible or elastically formed wall of the drip chamber. The process of venting by cyclical squeezing of the wall of the drip chamber prior to the start of an infusion is termed "initial pumping".

The region between the base of the drip chamber and the filter arranged in the latter may be vented without problem by cyclical squeezing the wall of the drip chamber beneath the filter. A suitable marking is preferably provided at the level of the drip chamber, up to which the liquid is to be initially pumped during priming of the infusion apparatus.

The filter may preferably be arranged at different locations in the infusion apparatus. However, attention should be paid, as far as possible, to the fact that the filter ought to take up the largest possible cross-sectional area within the infusion path, so that the flow resistance remains as small as possible.

Filters at the base of a drip chamber are basically known (WO 96/34565). They serve to keep latex particles, which are separated from the plug material on penetration of the lance into the plug of an infusion bottle, and other contaminants, which can enter into the liquid passage of the lance, away from the patient. The filter of the invention likewise satisfies this function, has, however, additionally the function of a block which avoids the complete emptying of the drip chamber.

The invention can also be realized with a known drip chamber of this kind, where the filter is provided at the base, in that an auxiliary drip chamber is sealingly arranged between the drip chamber and the tube. Through the spacing brought about in this way of the liquid filter provided in the base of the drip chamber from the tube, this embodiment also achieves a situation in which no air can enter into the tube on emptying of the infusion bottle, provided the auxiliary drip chamber was previously filled with sufficient infusion solution by initial pumping.

The mean pore size of the filter of the invention can be of the same order of magnitude as that of the known filter arrangement at the base of the drip chamber. Whereby the average pore size is preferred to be less than 20 µm, in particular less than 15 µm but larger than 5 µm. A preferred embodiment has an average pore size of 5 to 11µm. By reducing the pore size, the blocking action of the filter of the invention can be increased in a desired manner, however, on the other hand, the smaller the pore size is, the slower or impeded the flow of the liquid through the infusion apparatus will be, which generally is not desirable.

A further aspect of the present invention lies in a hydrophilic coating (formed for example by a plasma coating process) on the filter surface. By means of the hydrophilic coating of the filter the optimum force responsible for the build up of a liquid column in the drip chamber acting against gravity, and hence an acceptable height of deployment with respect to the patient may be achieved. The maximum column of liquid is achieved when the hydrostatic pressure equals the capillary pressure. The more uniform the hydrophilic coating is, the more effective the present invention is. Any defect in the coating will have the opposite effect. The hydrophilic coating preferably extends over the full cross section of the filter.
The mounting of the filter to the surrounding components is likewise important, so that the effect of preventing air from passing through the filter may be reliably ensured up to a desired height difference (pressure difference) between the infusion bottle and the patient's level. By mounting the filter to the surrounding components such that, when it is covered by a liquid film and the pores are filled with liquid, it does not come into contact with any other component or structural element of the infusion apparatus, it is accomplished that the filter will cause a blockage of the flow of liquid even at a height at which conventional infusion apparatuses are usually deployed in hospital environments.

Further the invention provide for a specific structure above the filter to facilitate the restarting of the flow of liquid by assisting in the displacement of liquid from the pores of the filter. The structure above the filter causes the force acting on the liquid to be used more efficiently by reducing the effective surface area the force is acting on. Hence a smaller force will achieve the desired result or in other words the initial pumping can take place without a recognizable increase in resistance in comparison to a standard infusion apparatus.

The invention will be described by way of example in the following with reference to the drawings, in which are shown:
Figures 1, 3 and 4 do not show all features of the invention.
- Fig. 1: a schematic, partly sectioned view of an infusion apparatus for illustrating principles of the invention,
- Fig. 2: a sectional view of only the drip chamber with the lancing part of a further embodiment and with the arrangement of the liquid filter in the lancing part,
- Fig. 3: a corresponding sectional view of an arrangement of the liquid filter in the liquid passage of the lance,
- Fig. 4: a corresponding sectional view, in which the drip chamber is extended at the bottom by an auxiliary drip chamber,
- Fig. 5: a sectional view analogous to Fig. 2 of an embodiment of the infusion apparatus of the invention provided with a guide plate, and
- Fig. 6: the section VI of Fig. 5 to a substantially increased scale.

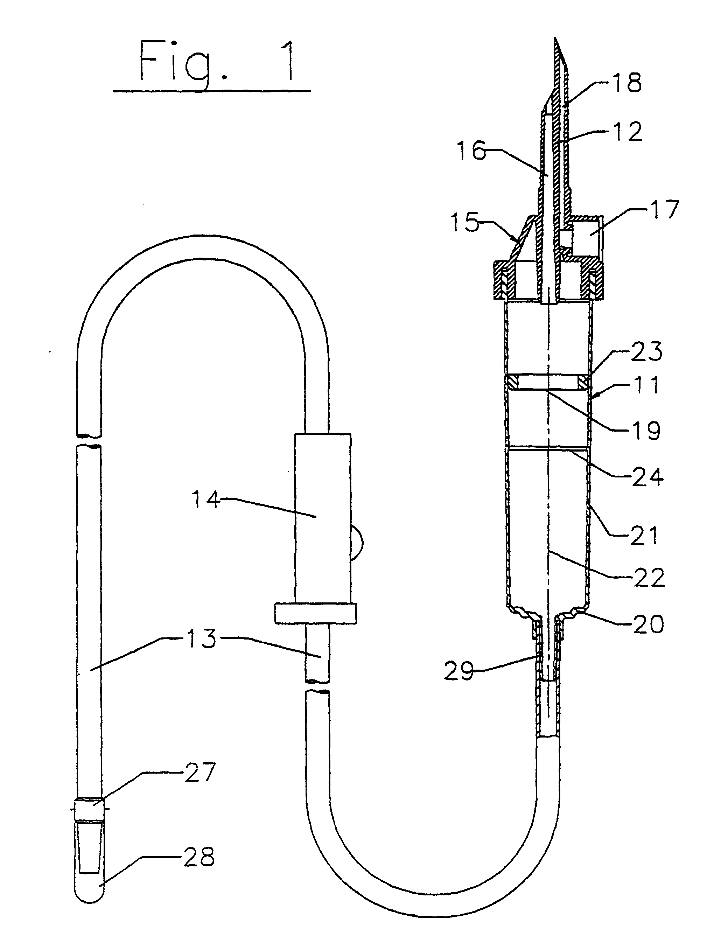

In accordance with Fig. 1, an infusion apparatus has a flexible and preferably also transparent tube, with a flow regulator 14 in the form of a tube clamp. At one end the tube has a connection piece 27 which is covered by a removable cap 28 and has an outer cone for the attachment of a non-illustrated canula. At the other end, the tube is sealingly pushed onto a connection stub 29 provided at the bottom of a drip chamber 11 having a resilient wall 21, in order to bring the tube into flow connection with the drip chamber 11.

A lancing portion 15 is sealingly mounted onto an upper open end of the drip chamber 11 and has a lance 12. The lancing portion 15 contains a liquid passage 16, the upper end of which opens into the surrounding atmosphere and the lower end of which opens in the interior of the drip chamber 11. An air passage 18 extends parallel to the liquid passage 16 in the lance 12. The air passage 18 opens into the surrounding atmosphere at the upper tip of the lance 12, preferably above the upper end of the liquid passage 16 and into the surrounding atmosphere in the rear region via an air filter 17. After the penetration of the lance 12 into a non-illustrated infusion bottle, i.e. the plug of the bottle, the upper ends of the passages 16, 18 are located in the interior of the infusion bottle, whereas the air filter 17 produces an air suction connection to the outer atmosphere.

A holding ring 23 is sealingly inserted in the upper region of the drip chamber 11 and a liquid filter 19, which is formed as a thin plate extending perpendicular to the longitudinal axis of the drip chamber 11, which preferably has a circular cross-section, is secured to the holding ring 23. A marking 24 is located between the base 20 of the drip chamber 11 and the filter 19, approximately at the mid-height of the drip chamber 11. The drip chamber 11 should consist of a transparent or at least translucent material, so that the liquid level within the drip chamber 11 can be checked from the outside.

The operation of the described infusion apparatus is as follows:
After the penetration of the lance 12 from the bottom into the plug of an infusion bottle suspended upside down, the initial pumping is first effected with the tube clamp 14 closed, such that the drip chamber 11 is vented between its base 20 and the liquid filter 19 by cyclical squeezing. Each time it is pressed together, air is pumped through the liquid channel 16 into the interior of the infusion bottle, whereupon, on subsequent release of the drip chamber 11, liquid is sucked in from the infusion bottle through the liquid passage 16 into the drip chamber 11. In this manner the liquid level in the drip chamber 11 rises in stages until it finally reaches the marking 24. Once this is achieved, the tube 13 is filled fully with liquid by letting out a part of the infusion solution with the tube clamp open. The tube clamp 14 is now closed again. The infusion apparatus is now ready for operation, i.e. the cap 28 can be withdrawn from the connection piece 27, and the connection piece 27 can be mounted onto a canula, which has been pushed into the patient's vein.

Thereafter, with renewed opening of the tube clamp 14, the infusion bottle is permitted to empty, with air being sucked in through the air filter 17 and the air passage 18, so that at least substantially atmospheric pressure always prevails in the infusion bottle above the infusion solution.

The infusion liquid will flow through the liquid passage 16 downstream into the drip chamber 11 passing through the filter 19. When the infusion bottle is empty, liquid will remain in the pores of the filter 19. This liquid held in the pores of the filter 19 will prevent any air from passing through the filter and entering the drip chamber 11, thereby creating a seal at the upper end of the drip chamber 11.

Due to the sealing effect of filter 19, a liquid column will be maintained in the drip chamber 11 as the liquid in the drip chamber will be held by the capillary pressure which is acting against gravity. An amount of liquid will remain in the drip chamber as long as the pores of the filter 19 are covered by a liquid coating, thereby preventing air from entering the drip chamber. The atmospheric pressure only acts in a reduced manner on the liquid located in the lower part of the drip chamber 11 as a result of the blocking action of the filter 19 in accordance with the invention. The maximum column of liquid in the drip chamber 11 is achieved when the hydrostatic pressure equals the capillary pressure.

The empty infusion bottle may now be removed and replaced by a full infusion bottle. When the full infusion bottle has been placed on the lancing part 15 of the infusion apparatus, the liquid in the bottle will be prevented from flowing through the liquid passage 16 to the filter 19 by the air which is trapped between the filter 19, which acts as a seal, and by the liquid in the bottle which is forced into the liquid passage 16 as a result of gravity.

In order to restart the flow of the liquid into the drip chamber 11, it is preferable to apply a pumping action by squeezing the flexible side wall of the dripping chamber 11. The squeezing action increases the pressure in the drip chamber 11 by reducing the volume of the drip chamber causing the air in the section of the dripping chamber which is not filled with the infusion liquid to be forced upwards towards the filter and consequently causing the liquid located in the pores of the filter 19 to be pushed through the filter 19. Thereby, at least some of the liquid is removed from the pores. If the force of the air pushed upward is sufficient enough to displace the liquid covering the pores of the filter, then the sealing effect of the filter covered with liquid is broken. Air may then enter into the drip chamber 11. The liquid in the full infusion bottle will, now no longer being blocked by the air in the liquid passage 16, flow through the filter and on via the tube to the patient. The air which prevented the liquid from flowing will be forced into the infusion bottle such that the infusion bottle is allowed to run empty as described above. The sealing properties of the filter 19 are determined by its hydrophilic properties and the specific pore size.

In the following figures, the same reference numerals designate corresponding components to tube in Fig. 1.

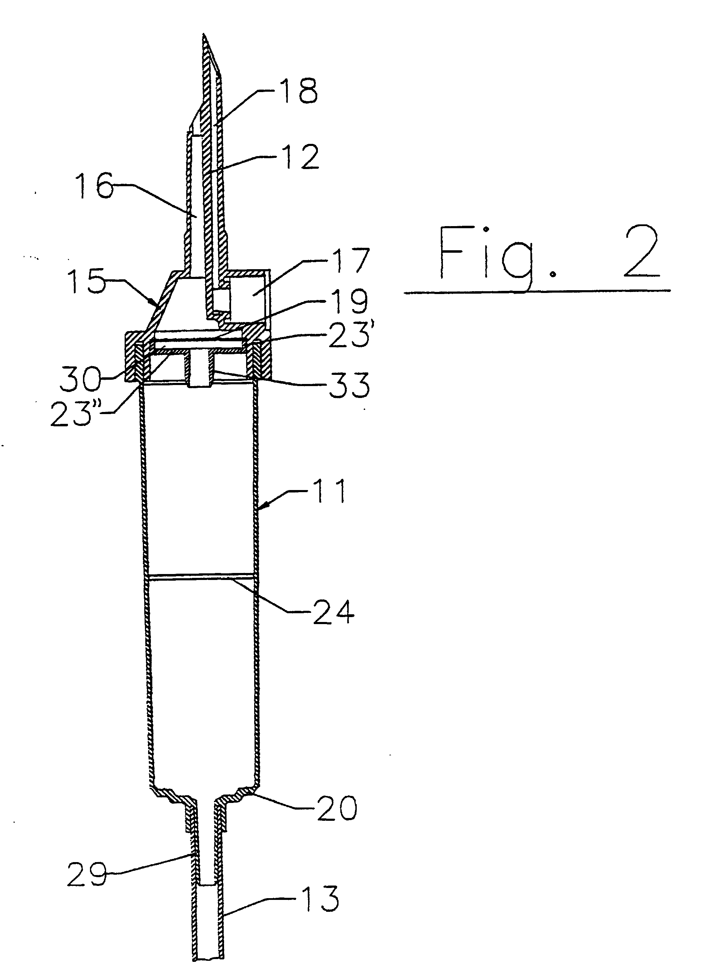

In accordance with Fig. 2, the liquid filter 19 is arranged in a space 30 especially provided for it in the lower region of the lancing part 15. The space has a cross-section which it is at least approximately as large as that of the drip chamber 11, which provides for a low flow resistance of the liquid filter 19. By arranging the liquid filter 19 in the lancing part 15 the total height of the drip chamber 11 is available for the reception of liquid during initial pumping and subsequent operation. For the mounting of the filter 19 a sealingly mounted holding ring 23' comprising washer-like base 23" and a central supply stub 33, is provided at the lower part of the lancing part 15.

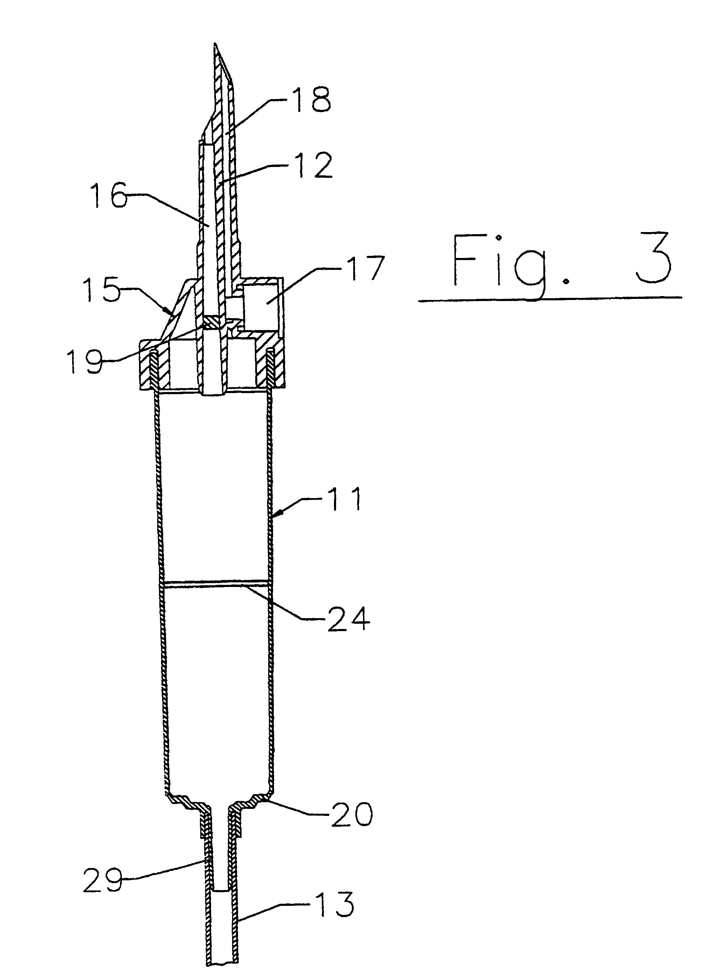

In the embodiment of Fig. 3 the liquid filter 19 is arranged within the liquid passage 16 of the lance 12, which represents a particularly simple measure construction-wise, but leads to an increased flow resistance, so that the pore size of the liquid filter 19 in this case may not be made too small.

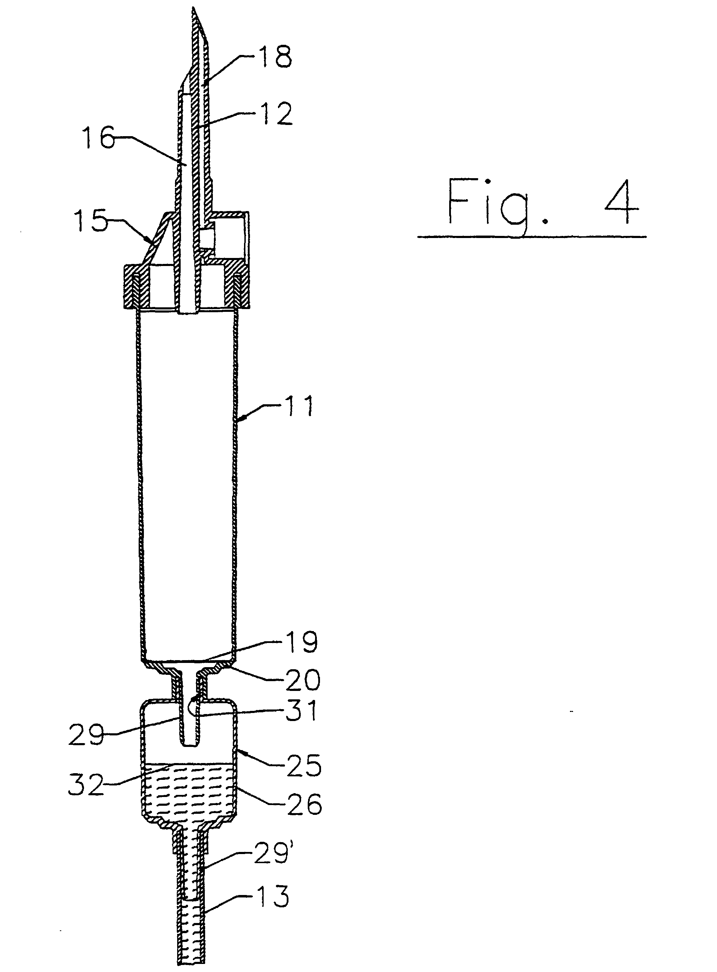

The embodiment of Fig. 4 shows how a liquid filter can also be realised with a known drip chamber 11, in which a liquid filter 19 is arranged at the base 20 of the drip chamber 11.

In this case one can sealingly place the upper opening 31 of an auxiliary drip chamber 25 onto the connection stub 29 of the drip chamber 11 from below, with the auxiliary drip chamber 25 having a connection stub 29' at the bottom, onto which the tube 13 is sealingly pushed. The auxiliary drip chamber 25 has an elastic wall 26, so that it can itself be initially pumped at the start of operation in analogous manner to the drip chamber 11, so that a desired liquid level 32 is achieved in the auxiliary drip chamber 25.

In this case the liquid filter 19 provided at the base 20 of the drip chamber 11 brings about a corresponding blocking action to that of the preceding embodiments, so that the drip chamber 11 cannot itself run fully empty via the auxiliary drip chamber 25, and thus the entry of air into the tube 13 is prevented, despite the arrangement of the liquid filter 19 at the base of the drip chamber 11.

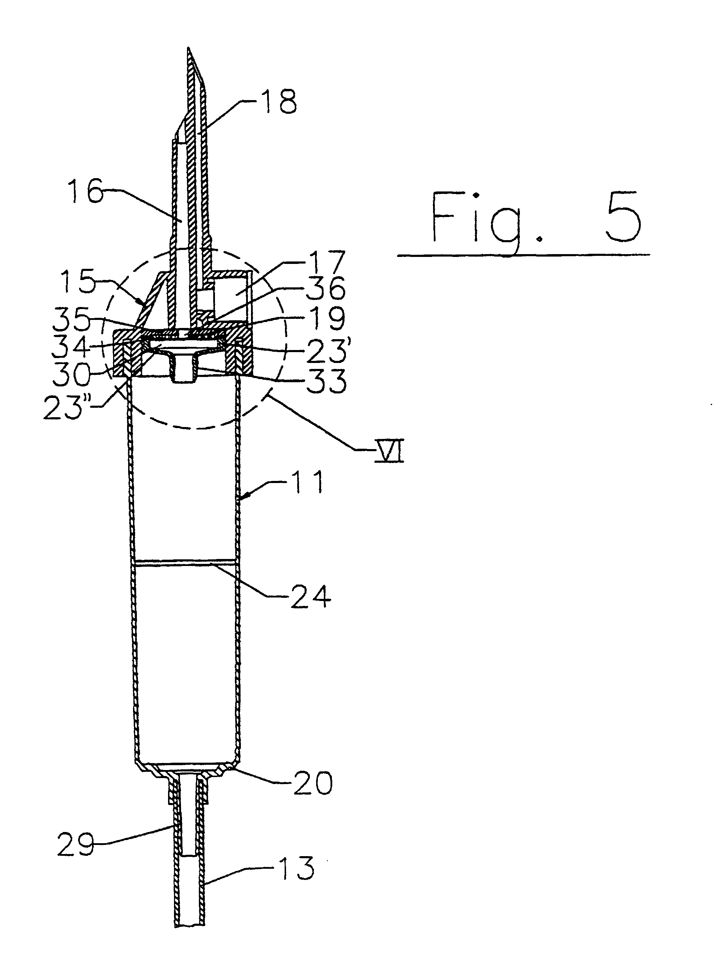

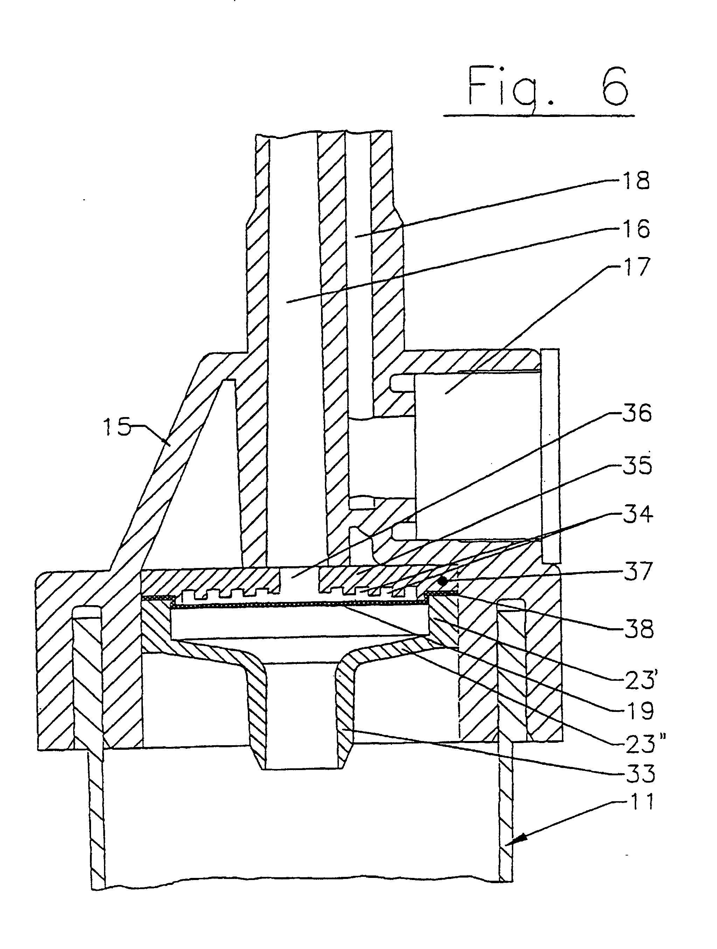

In the embodiment of Figs. 5 and 6 a substantially circular washer-like guide plate 35 with a central passage 36 and recesses 34 provided at its underside is arranged above the sheet-like liquid filter 19. The recesses 34 can have the form of grooves concentric or radial to the central passage 36. Preferably, the recesses are in flow communication with the central connection passage 36. As is shown in Fig. 6, the liquid filter 19 does not contact the guide plate 35 it its stationary position. Only when the filter 19 is forced upward, will it come into contact with the lower surface of the guide plate 35, so that the recesses 34, which are only open downwardly and are closed toward the top, likewise adjoin the upper surface of the liquid filter 19.

As a result of this design, the force necessary to displace the liquid covering the pores of the filter 19 is reduced, since only such pores which are located below a recess 34 and therefore do not come into contact with the lower surface of the guide plate 35 will be effected by the upward stream of air during the pumping action. Hence the person applying the infusion apparatus, the nurse or doctor, will not notice any change in application in comparison to conventional infusion apparatuses. Neither the flow of liquid through the filter 19, nor the function of the liquid filter 19 not to allow air to pass through it during the emptying of the infusion bottle is impaired.

The filter 19 is mounted in the infusion apparatus in such a manner that only the circumference of the filter 19 is fixed and that the inner region of the filter is not in contact with any other component or structural element of the infusion apparatus. In Fig. 6, rim 38 of the filter 19 is clamped between the holding ring 23 and the radially outwardly disposed mounting rim 37 of the guide plate 35. The connection of the filter 19 and the holding ring 23 and the mounting rim 37 preferable is fixed using a technique of ultrasound welding.

A further aspect of the present invention is a hydrophilic coating of the filter. The preferred filter material is either polyamide nylon 6.6, polyamide nylon 11 or polyester-polyethylene teraphthalate. The hydrophilic coating preferably is applied in accordance with a plasma coating process.

## Claims

1. An infusion apparatus comprising a drip chamber (11) having flexible walls (21) for venting the drip chamber (11), a tube (13) leading from an outlet end of the drip chamber (11), a lancing part (15) sealingly mounted at an inlet end of the drip chamber (11) and having a lance (12) for puncturing the plug of an infusion bottle, a liquid passage (16) extending through the lance (12) and opening at one end in the drip chamber and at the other end in a front region of the lance (12), wherein a liquid filter (19) is mounted on its periphery in the path of the infusion solution from the lance (12) to the tube (13) above the base (20) of the drip chamber (11), thereby occupying the entire cross-section available for fluid flow, the filter (19) having a hydrophilic coating and a plurality of pores, wherein the average size of the pores lies between 5 and 20 µm,
**characterized in that**
the infusion apparatus further comprises means (35) located above the filter (19) for reducing the force necessary for displacing the infusion solution from some of the pores of the filter (19)in an upward direction during venting.

2. The infusion apparatus according to claim 1, **characterized in that** the means (35) are formed by a guide plate (35) having an opening (36) and recesses (34) formed therein such that the recesses (34) are in flow communication with the liquid passage (16).

3. The infusion apparatus according to claim 2, **characterized in that** the guide plate (35) is positioned above the filter (19), and **in that** the filter (19) comes in contact with a lower surface of the guide plate (35) when the filter (19) is forced upwardly by the pressure generated in the drip chamber due to squeezing of the sidewalls, wherein the pressure is applied to displace the infusion solution form the pores of the filter (19).

4. Infusion apparatus according to claim 2 or 3, **characterized in that** at least the part of recesses (34) adjoining the liquid filter (19) are dimensionally at least an order of magnitude greater than the pores of the liquid filter (19).

5. Infusion apparatus according to one of the claims 2, 3 or 4, **characterized in that** the recesses (34) have the form of spaced grooves that preferably extend concentrically and/or radially to the central passage (36).

6. Infusion apparatus according to one of the claims 2 to 5, **characterized in that** the recesses (34) are only open to the liquid filter (19) and to the connection passage (36), but are in other respects closed off towards the outside.

7. Infusion apparatus according to one of the preceding claims, **characterized in that** the liquid filter is mounted on a holding ring (23) that is sealingly inserted into the drip chamber (11).

8. Infusion apparatus according to claim 6, **characterized in that** the guide plate (35) has an extent corresponding to the liquid filter (19) and an attachment rim (37) that is in particular mounted onto a holding ring (23') with a filter rim (38) between them.

9. Infusion apparatus according to one of the preceding claims, **characterized in that** the liquid filter (19) is arranged in the lancing part (15).

## Patentansprüche

1. Ein Infusionsgerät aufweisend eine Tropfkammer (11) mit elastischen Wänden (21) zum Entlüften der Tropfkammer (11), einen Schlauch (13), der vom Auslassende der Tropfkammer (11) führt, ein am Einlassende der Tropfkammer (11) dicht angebrachtes Einstechteil (15) mit einem Einstechdorn (12) zum Durchstechen des Infusionsflaschenverschlusses, einen sich durch den Einstechdorn (12) erstreckenden Flüssigkeitskanal (16), der an einem Ende in der Tropfkammer (11) und an dem anderen Ende im vorderen Bereich des Einstechdorns (12) mündet, wobei ein Flüssigkeitsfilter (19) an dessen Umfang im Pfad der Infusionslösung aus dem Einstechdorn (12) zum Schlauch (13) oberhalb des Bodens (20) der Tropfkammer (11) befestigt ist, wodurch der gesamte für den Flüssigkeitsfluss zur Verfügung stehende Querschnitt eingenommen wird, wobei das Filter (19) eine hydrophile Beschichtung und eine Vielzahl von Poren mit einer mittleren Porengröße von 5 bis 20 µm aufweist,
**dadurch gekennzeichnet, dass**
das Infusionsgerät ferner Mittel (35) aufweist, die oberhalb des Filters (19) angeordnet sind, zur Reduzierung der Kraft, die während der Entlüftung für die Verdrängung der Infusionslösung aus einigen Poren des Filters (19) in eine Aufwärtsrichtung erforderlich ist.

2. Infusionsgerät nach Anspruch 1, **dadurch gekennzeichnet, dass** die Mittel (35) durch eine Leitplatte (35) mit Öffnungen (36) und darin so geformte Ausnehmungen (34), dass die Ausnehmungen (34)in Strömungsverbindung mit dem Flüssigkeitskanal (16) stehen.

3. Infusionsgerät nach Anspruch 2, **dadurch gekennzeichnet, dass** die Leitplatte (35) oberhalb des Filters (19) angeordnet ist, und dass der Filter (19) in Berührung mit einer unteren Oberfläche der Leitplatte (35) kommt, wenn das Filter (19) durch den in der Tropfkammer durch Zusammendrücken der Seitenwände erzeugte Druck nach oben gedrängt wird, wobei der Druck zum Verdrängen der Infusionslösung aus den Poren des Filters (19) aufgebracht wird.

4. Infusionsgerät nach einem der Ansprüche 2 oder 3, **dadurch gekennzeichnet, dass** zumindest der an das Flüssigkeitsfilter (19) angrenzende Teil der Ausnehmungen (34) dimensionsmäßig um zumindest eine Größenordnung ausgedehnter als die Poren des Flüssigkeitsfilters (19) ist.

5. Infusionsgerät nach einem der Ansprüche 2, 3 oder 4, **dadurch gekennzeichnet, dass** die Ausnehmungen (34) die Form von beabstandeten Rillen haben, die vorzugsweise konzentrisch und/oder radial zum Zentralkanal (36) verlaufen.

6. Infusionsgerät nach einem der Ansprüche 2 bis 5, **dadurch gekennzeichnet, dass** die Ausnehmungen (34) nur zum Flüssigkeitsfilter (19) und zum Verbindungskanal (36) offen, und im Übrigen nach außen geschlossen sind.

7. Infusionsgerät nach einem der vorhergehenden Ansprüche, **dadurch gekennzeichnet, dass** das Flüssigkeitsfilter (19) an einem Haltering (23) angebracht ist, der dicht in die Tropfkammer (11) eingesetzt ist.

8. Infusionsgerät nach einem der vorhergehenden Ansprüche, **dadurch gekennzeichnet, dass** die Leitplatte (35) einer Ausdehnung entsprechend dem Flüssigkeitsfilter (19) aufweist und einen Befestigungsrand (37) besitzt, der unter Zwischenschaltung eines Filterrands (38) insbesondere auf einen Haltering (23') aufgesetzt ist.

9. Infusionsgerät nach einem der vorhergehenden Ansprüche, **dadurch gekennzeichnet, dass** das Flüssigkeitsfilter (19) in dem Einstechteil (15) angeordnet ist.

## Revendications

1. Infuseur comprenant une chambre goutte à goutte (11) possédant des parois latérales (21) flexibles pour permettre la purge de la chambre (11), un tube (13) s'étendant d'une extrémité de sortie de la chambre goutte à goutte (11), une partie de perforation (15) montée de façon étanche sur une extrémité d'entrée de la chambre goutte à goutte (11) et comprenant un perforateur (12) destiné à perforer le bouchon d'un flacon de produit à infuser, un passage de liquide (16) s'étendant à l'intérieur du perforateur (12) et s'ouvrant à une extrémité dans la chambre goutte à goutte et à l'autre extrémité dans une région antérieure du perforateur (12), dans lequel un filtre à liquide (19) est monté sur sa périphérie dans le trajet parcouru par la solution à infuser entre le perforateur (12) et le tube (13), au-dessus du fond (20) de la chambre goutte à goutte (11), occupant ainsi l'intégralité de la section offerte pour l'écoulement du liquide, le filtre (19) présentant un revêtement hydrophile et une pluralité de pores, la taille moyenne des pores étant comprise entre 5 et 20 µm, **caractérisé en ce que** l'infuseur comprend également un moyen (35), disposé au-dessus du filtre (19), destiné à réduire la force nécessaire pour chasser la solution à infuser de certains des pores du filtre (19) vers le haut pendant l'opération de purge.

2. Infuseur selon la revendication 1, **caractérisé en ce que** le moyen (35) est constitué d'une plaque de guidage (35) présentant une ouverture (36) et des cavités (34) formées dans celle-ci de telle manière que les cavités (34) sont en communication de fluide avec le passage de liquide (16).

3. Infuseur selon la revendication 2, **caractérisé en ce que** la plaque de guidage (35) est positionnée au-dessus du filtre (19) et **en ce que** le filtre (19) entre en contact avec la face inférieure de la plaque de guidage (35) lorsque le filtre (19) est poussé vers le haut par la pression engendrée dans la chambre goutte à goutte par l'écrasement des parois latérales, cette pression étant appliquée pour chasser la solution à infuser des pores du filtre (19).

4. Infuseur selon la revendication 2 ou 3, **caractérisé en ce qu'**au moins la partie des cavités (34) adjacentes au filtre à liquide (19) est dimensionnée avec une taille supérieure d'au moins un ordre de grandeur à celle des pores du filtre à liquide (19).

5. Infuseur selon l'une des revendications 2, 3 ou 4, **caractérisé en ce que** les cavités (34) ont la forme de rainures espacées, s'étendant de préférence concentrique-ment et/ou radialement par rapport au passage central (36).

6. Infuseur selon l'une des revendications 2 à 5, **caractérisé en ce que** les cavités (34) sont seulement ouvertes vers le filtre à liquide (19) et vers le passage de connexion (36), mais sont sinon fermées vers l'extérieur.

7. Infuseur selon l'une des revendications précédentes, **caractérisé en ce que** le filtre à liquide est monté sur une monture annulaire (23) qui est insérée de manière étanche dans la chambre goutte à goutte (11).

8. Infuseur selon la revendication 6, **caractérisé en ce que** la plaque de guidage (35) présente une extension qui correspond au filtre à liquide (19) et un collet de fixation (37) qui est en particulier monté sur une monture annulaire (23') avec un collet de filtre (38) entre les deux.

9. Infuseur selon l'une des revendications précédentes, **caractérisé en ce que** le filtre à liquide (19) est disposé dans la partie de perforation (15).
